(19) Europäisches Patentamt
European Patent Office
Office européen des brevets

(11) Publication number: **0 131 561**
**B1**

(12) # EUROPEAN PATENT SPECIFICATION

(45) Date of publication of patent specification: **15.10.86**

(51) Int. Cl.$^4$: **C 07 C 17/38,** C 07 C 19/02

(21) Application number: **84870094.4**

(22) Date of filing: **04.07.84**

(54) **Process for producing monoadducts of olefins and telogens reactive therewith.**

(30) Priority: **06.07.83 US 511130**

(43) Date of publication of application:
**16.01.85 Bulletin 85/03**

(45) Publication of the grant of the patent:
**15.10.86 Bulletin 86/42**

(84) Designated Contracting States:
**AT BE CH DE FR GB IT LI LU NL**

(56) References cited:
**US-A-2 658 930**
**US-A-4 243 607**

(73) Proprietor: **MONSANTO COMPANY**
**Patent Department 800 North Lindbergh Boulevard**
**St. Louis, Missouri 63166 (US)**

(72) Inventor: **Woodard, Scott Santford**
**1208 Cottage Mill Drive**
**Ballwinn Missouri 63011 (US)**

(74) Representative: **Lunt, John Cooper et al**
**Monsanto Europe S.A. Patent Department**
**Avenue de Tervuren 270-272 Letter Box No 1**
**B-1150 Brussels (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 131 561 B1

**Description**

Background of the invention

This invention relates to the field of telomerization reactions of olefinic and acetylenic taxogens and, more particularly, to an improved process for preparing a monoadduct of a taxogen and a telegon by reaction in the presence of iron and a promoter for the reaction.

Addition-polymerizable olefinically unsaturated monomers are known to react with certain compounds, refered to as telegons, by a process known as telomerization, in which the telogen splits to form two monovalent terminating groups and the product contains a number of units derived from the monomer, capped on each end with moieties derived from splitting of the telegon. More particularly, there are known processes in which carbon tetrachloride acts as a telogen for the telomerization of ethylene according to the general equation:

$$CCl_4 + nCH_2{=}CH_2 \rightarrow Cl{-}C(Cl)(Cl){-}(CH_2{-}CH_2)_n{-}Cl \qquad \text{(Eq. 1)}$$

where n is an integer such that the molecular weight of the telomerization product is relatively low compared to the molecular weight typical of high polymers. The species where n equals 1, i.e., 1,1,1,3-tetrachloropropane is an intermediate useful for the manufacture of a variety of end products, including agricultural chemicals, pharmaceuticals, gums, silicons and the like. Accordingly, there has been a need in the art for processes by which the above reaction can be controlled to produce the 1,1,1,3-product in high yield and with high productivity, minimizing both the formation of telomerization products in which n2 and the formation of various other reaction side products such as, for example, 1,3,3,5-tetrachloropentane. The latter by-product is formed when 1,1,1,3-tetrachloropropane itself serves as a telogen in reaction with ethylene.

Illustrative art relating to the reactions of carbon tetrahalides and olefins includes Thompson U.S. patent 2,658,930, Nakagawa et al. U.S. patent 3,631,115, Decker et al. U.S. patent 3,454,657, Asscher et al. U.S. patent 3,651,019, Takamizawa et al. U.S. patent 4,243,607 and Asahara et al. "The Telomerization of Carbon Tetrachloride and Ethylene" Kogyo Kagaku Zasshi 1971, 74(4), 703—705.

Thompson discloses reactions of olefins with carbon tetrachloride in the presence of metallic iron, water and oxygen. Yields reported by Thompson are relatively low while reaction times are very extended, Example 1 involving storage of the reactants at 38°C for over three weeks.

Nakagawa et al. disclose telomerization of an alkene taxogen with a halogenoalkane telogen in the presence of a nickel peroxide catalyst. The monomers which form the units between the capping moieties of a telomer are generically referred to in the art as taxogens.

Decker et al. describe catalytic synthesis of organic halogen compounds by reacting an ethylenically unsaturated compound with carbon tetrachloride or another halogenated compound in the presence of an amine compound and copper metal or copper salt.

Asscher et al. note that the use of free radical catalysts such as peroxides tends to cause the ethylene-carbon tetrachloride telomerization reaction to progress to the extent that substantial portions of products in which $n \geq 2$ appear in the reaction product, even where the ratio of carbon tetrachloride to olefinically unsaturated material employed is as high as 100:1. Asscher et al. describe a system in which 1:1 adducts of carbon tetrachloride and olefinically unsaturated compounds are produced in a solvent system using a catalyst consisting of a dissolved copper or iron compound. Asscher et al. note that it is essential to have ferrous iron present and disclose various reducing agents which may be incorporated in the reaction system for such purpose. According to the Asscher et al. disclosure, a special reducing agent may not be necessary in all systems since olefins and/or certain solvents may serve the function of maintaining a portion of the iron in the ferrous state.

Asahara et al. describe the reaction of carbon tetrachloride and ethylene in the presence of a catalyst system consisting of a trialkyl phosphite and ferric chloride hexahydrate. Asahara et al. succeed in achieving relatively rapid reaction but obtain a product containing a substantial fraction of by-products, including higher telomerization products ($n \geq 2$) and tars.

Takamizawa et al. disclose an improvement on the Asahara system in which an olefin is reacted with carbon tetrachloride in the presence of a three-component catalyst system including ferric chloride hexahydrate, a trialkyl phosphite or diethyl phosphite, and a nitrile such as acetonitrile. This catalyst system enables Takamizawa et al. to obtain a reasonably high yield of product in which $n=1$ while reducing, but not eliminating, the formation of by-product tars as compared to Asahara. Although they did not ascribe any particular significance to it, both Asahara and Takamizawa et al. used stainless steel autoclaves for carrying out their reactions.

Despite the various efforts that have been made in the art to develop effective reaction systems for producing monoadducts of olefins and carbon tetrahalides, a need has remained for a system which

eliminates tars and produces then =1 product in both high selectivity and high yield at a high rate of production.

Summary of the invention

Briefly, the present invention is directed to a process for producing a monoadduct of a taxogen and a telogen reactive therewith. In this process, the telogen is reacted with the taxogen in the presence of both a promoter and a source of metallic iron that is effective as an activator for the reaction. The promotor comprises a phosphorus (V) compound that contains a phosphoryl group.

The invention is further directed to a process for preparing a monoadduct of a taxogen and a telogen reactive therewith, while substantially avoiding the formation of higher telomerization products. The process comprises reacting the telogen and the taxogen in a reaction system comprising a liquid phase and a source of metallic iron in contact with the liquid phase, the source of metallic iron being effective as an activator for the reaction. The liquid phase comprises the telogen and a promoter for the reaction. The promoter comprises a phosphorus (V) compound that contains a phosphoryl group and is compatible with the telogen.

Description of the preferred embodiments

In accordance with the present invention, it has been discovered that high yield, selectivity and productivity in the preparation of monoadducts of taxogens and telogens reactive therewith are achieved by carrying out the reaction in the presence of an active source of metallic iron and a promoter which comprises a phosphorus (V) compound containing a phosphoryl group. The reaction system for preparing the monoadduct includes a liquid phase that contains the telogen and is in contact with the source of metallic iron. The phosphorus compound, and preferably also a ferric salt, are dissolved in the liquid phase. Ferric salt may be either added as such to the system or generated *in situ* prior to commencement of the telomerization reaction. By use of such reaction systems, formation of higher telomerization products, tars and other unwanted by-products is substantially eliminated and the reaction product is essentially a single phase liquid. Where the telogen is an alkyl halide such as a haloform or carbon tetrahalide, the reaction may optimally be carried out to nearly 100% conversion of the telogen. Often, the product monoadduct can be removed from the reactor and be used directly with little or no purification as an intermediate for the production of other products. Where the reaction mixture contains any unreacted material after the reaction is completed, the unreacted material can be removed by distillation.

Although the instant invention is defined only by the appended claims and is not limited to a particular theory, it is believed that the reaction is initiated by a redox transfer between telogen and ferrous ion which splits the telogen to form a free radical and a ligand which is attached to the ferric ion produced in the transfer. Thus, in the case of alkyl halide, a halide ion ligand is thought to combine with the ferrous ion in accordance with the following equation:

$$Fe^{++}+RX \rightarrow Fe^{+++}+R\cdot \qquad \text{(Eq. 2)}$$

where R is an alkyl group and X is halogen.

According to this theory, the taxogen adds to the free radical producing another free radical that in turn undergoes a redox transfer with the ferric ion, recovering therefrom the halide ligand which forms an end cap for the telomerization product. For example, in the case of ethylene:

$$nCH_2=CH_2+R\cdot \rightarrow R(CH_2-CH_2)_n\cdot \qquad \text{(Eq. 3)}$$

$$R(CH_2-CH_2)_n\cdot+FeX^{++} \rightarrow R(CH_2-CH_2)_nX+Fe^{++} \qquad \text{(Eq. 4)}$$

The cycle is then thought to be repeated by reaction of the regenerated ferrous ion with the telogen in accordance with Equation 2.

Whatever the mechanism, it has been found that the presence of a source of metallic iron, which is capable of being an activator for the reaction, is essential to the progress of the reaction. This contrasts with the teachings of the Asahara et al. and Takamizawa et al. references which teach only the incorporation of a ferric salt in the reaction system. While the reactions of the Asahara et al. and Takamizawa et al. references were carried out in a stainless steel autoclave, the references do not reflect any appreciation that the presence of stainless steel may have contributed to the reaction. However, it has now been discovered that the unique combination of an active source of metallic iron and a phosphorus (V) compound containing a phosphoryl group provides high productivity, high selectivity and high yields of the monoadduct.

A very wide variety of olefins and alkynes can be used as the taxogen in the process of the invention. Most advantageously, the process can be used to produce monoadducts of olefinic hydrocarbons such as ethylene, propylene, 1-butene, 2-butene, 1-hexene, 3-hexene, 2-octene, 1-dodecene, 4-dodecene, isoprene, 3-methyl-5-heptene, 1-phenyl-3-hexene, butadiene, vinyl halides and the like. However, other substituted olefins such as acrylic esters, acrylamide, acrylonitrile, acrolein, vinyl esters, vinyl ethers, vinyl silanes, cinnamic acid, cinnamaldehyde, etc., can also be used. In a further advantageous embodiment of the

invention, the taxogen may be a polymer containing unsaturated carbon to carbon bonds, for example, a natural or synthetic rubber, and the telomerization product may be a halogenated polymer.

Preferably, the non-polymeric olefinic taxogens correspond to the formula:

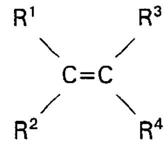

where each of $R^1$, $R^2$, $R^3$, and $R^4$ may be independently selected from among hydrogen, alkyl, aryl, alkenyl, and alkynyl, either unsubstituted or substituted with substituents which are compatible with the reaction which forms the desired monoadduct. Such substituents are considered so compatible if they do not inhibit the desired reaction or cause competitive side reactions which proceed preferentially to the desired reaction under the prevailing reaction conditions. Other groups meeting the same compatibility criterion can also constitute $R^1$, $R^2$, $R^3$ and $R^4$. These include cyano, halogen, imido, carbonyl, alkoxy, aryloxy, alkenoxy, acyloxy, silyl, and phosphoryl. Substituents containing carbon preferably have 1 to about 100 carbon atoms.

Useful alkynes include those corresponding to the formula

$$R^5—C \equiv C—R^6$$

when $R^5$ and $R^6$ are independently selected from among the same substituents that may comprise $R^1$ to $R^4$.

Preferably, the telogen used in the reaction of the invention is an alkyl halide having 1 to about 100 carbon atoms. The process of the invention is especially suited to the preparation of adducts of alphaolefins and carbon tetrahalides such as carbon tetrachloride, carbon tetrabromide, trichlorobromomethane, dichlorodibromomethane and chlorotrifluoromethane. Use of such telogens provides an advantageous route to the introduction of the trihalomethyl groups into a variety of organic structures. Moreover, once a tribromo- or trichloromethyl group is introduced, the corresponding trifluoromethyl compound may be readily produced by reaction of the telomerization product with hydrogen fluoride in the presence of antimony trifluoride.

Among the other telogens which can be used in the process are haloforms such as chloroform and bromoform, as well as a variety of other alkyl halides corresponding to the formula

$$R'X$$

Where R' is a substituted or unsubstituted alkyl group and X is a halogen. For rapid reaction and high yields, the alkyl halide telogen preferably contains a trihalomethyl or terminal dihalomethyl group.

It has been found that the use of a phosphorus (V) compound containing a phosphoryl group, in the presence of an active source of metallic iron, as the reaction promotion system provides significant advantages in yield, productivity or both as compared to previously known systems which use a trialkyl phosphite type catalyst. A particularly preferred class of phosphorus compounds includes alkyl and aryl phosphate esters, phosphoryl halides, phosphorus pentoxide and those phosphoryl compounds having the structure

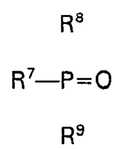

where $R^7$ is a substituted or unsubstituted hydrocarbyl group. For example, $R^7$ may be alkyl, aryl, or alkenyl, either unsubstituted or substituted with hydroxyl, carbonyl, sulfate, amino, or halo substituents. Particular groups which may constitute $R^7$ include methyl, ethyl, propyl, octyl, vinyl, allyl, phenyl, chloroethyl, butyl-amino, and the like. $R^8$ and $R^9$ are independently selected from among hydroxy, alkoxy, aryloxy, and the various groups which may comprise $R^7$. As noted, the phosphorus compound is contained in the liquid phase and should, therefore, be compatible with the telogen, i.e., soluble therein at least to the extent necessary to serve as a promoter for the reaction.

Exemplary phosphorus compounds suitable for use in the process of the invention include alkyl and aryl phosphate esters such as, for example, triethyl phosphate, tributyl phosphate, phenyl diethyl phosphate, diethyl phosphate, dibutyl phosphate, phenyl phosphate, butyl phosphate and the like. Other suitable phosphorus compounds include alkyl and aryl phosphonate esters such as dimethyl methylphosphonate, dimethyl ethylphosphonate, diethyl phenylphosphonate, phenyl ethyl methylphosphonate, diethyl vinylphosphonate, butyl butylphosphonate, triethyl phosphonoacetate, and the like. Still further suitable phosphorus (V) compounds include butylphosphonic acid, hexamethyl

phosphoramide, phosphoryl chloride and phosphorus pentoxide. It is believed that the phosphorus compound serves to promote oxidation and dissolution of metallic iron to produce ferrous ions in the liquid phase. Such dissolved ferrous ions are thought to be involved in the initiating step of the reaction mechanism per equation 2, supra. While the exact role of the phosphorus compound in such postulated mechanism is not understood, it has been discovered that the presence of the phosphorus (V) compound has a most advantageous effect on productivity and yield. In an effective range of concentration, the reaction of carbon tetrachloride and ethylene is approximately first order with respect to concentration of free phosphorus compound as defined hereinbelow.

The source of metallic iron used in the reaction system may comprise any ferrous metal which serves as an activator for the reaction. In the context of this disclosure, the term "activator" is thus defined as a ferrous metal whose presence causes the telomerization reaction to proceed at a rate higher than the reaction rate obtained in an otherwise identical reaction system from which the activator is absent. Generally effective activation is associated with corrosion of the metallic iron source to the extent of a measurable loss of weight over the course of the reaction.

Materials such as cabron steel, mild steel, cast iron and wrought iron are advantageously used as the source of metallic iron in the process. Certain stainless steels can also be used but are less preferred. Some ferrous alloys, including various stainless steels, have not been found to be effective.

It has been found that the most rapid reaction is achieved where there is a relatively high area of contact between the source of metallic iron and the liquid phase medium. The reaction is approximately first order with respect to the contact surface between the liquid phase and the source of metallic iron. Forms of iron such as screens, powder, filings, shavings, bars, sheets, steel wool, or tubes can be used. Screens, powder and filings afford a particularly high specific surface area. Most of these iron sources can be re-used in a series of batch reactions. However, in the case of cast iron, a scale remains on the surface as the iron dissolves which may inhibit successive batch reactions.

In carrying out the process of the invention, a reactor is charged with a liquid phase reactant, the phosphorus (V) compound and a source of iron, for example, in the form of iron bars, an iron screen or iron filings. Normally, the telogen is a liquid phase material at ambient conditions and the phosphorus (V) compound is dissolved therein, preferably in a proportion of between about 0.01 and about 40 mole percent based on the telogen charge, more preferably 0.1% to 20%. For the case of carbon tetrachloride and ethylene, the concentration of phosphorus compound is typically 0.05 to 20 mole percent, preferably 0.1 to 5.0 mole percent. When the taxogen is liquid, some or all of this reactant may be contained in the initial charge also, the proportion depending on the relative reactivities of taxogen and telogen. Where the taxogen is highly reactive, it is preferable to maintain an excess of telogen during the greater part of the reaction period in order to minimize formation of $n \geq 2$ telomerization products.

Reaction is initiated either by bringing the system containing both taxogen and telogen to reaction temperature, or by adding one reactant to a preheated system containing the other. In the case of a gaseous and/or highly reactive taxogen, the reaction system may initially contain a substantial excess of telogen and, in this instance, the reaction is caused to progress by introducing taxogen into the reaction mixture while maintaining it at the aforesaid temperature. Preferably, such taxogen is introduced at a controlled rate less than or equal to the rate of reaction. By this means, an excess of telogen is present essentially throughout the reaction, thereby minimizing the formation of telomerization products in which $n \geq 2$. Because of the flammability of many olefins and alkynes, air is preferably excluded from the reactor by operating under conditions where reactant pressure exceeds atmospheric, or by use of an inert gas blanket.

Where the taxogen is a gas, as in the case of reaction of ethylene, propylene, or acetylene with carbon tetrachloride, the reactor containing the telogen, phosphorus (V) compound, and source of iron is pressurized with taxogen, and the reaction proceeds by absorption of taxogen into the liquid phase. Prior to pressurization with taxogen, the reactor is purged with an inert gas to displace oxygen. Optionally, the reactor may then be purged with taxogen to displace inert gas.

In order to minimize the formation of undesired by-products where $n \geq 2$, the initial reactor charge preferably contains a ferric salt dissolved in the telogen in a proportion of between 0 and about 10 mole percent, the amount depending on the reactivity of the taxogen and its tendency to form higher telomerization products. The ferric salt, most preferably ferric chloride, can be added to the telogen either directly or in an organic solvent solution. Alternatively, the ferric salt may be generated *in situ* by heating a reaction system containing an alkyl halide telogen, phosphorus compound and source of metallic iron at a temperature and for a time adequate to inhibit formation of higher telomerization products. Conveniently, *in situ* generation of ferric salt can be carried out at a temperature of 50°C or higher, preferably near the reaction temperature.

Thus, including the case where no initial ferric salt is charged to the system, there are three alternative protocols for initiating the reaction. These are summarized below in Table 1.

TABLE 1

| Case | Taxogen initially present | Hold time prior to taxogen charge | $Fe^{+++}$ initially charged |
|---|---|---|---|
| 1 | Yes | No | Yes |
| 2 | Yes | No | No |
| 3 | No | Yes | No |

Although ferric iron is believed to be ultimately generated in case 2 as well as cases 1 and 3, the initial concentration may be insufficient to prevent formation of significant amounts of $n \geq 2$ by-products during the early stages of the reaction. Thus, cases 1 and 3 are preferred, case 3 being advantageous in avoiding the necessity for handling ferric salt and adding it as such as to the reactor.

As indicated above, a relatively high ratio of ferric ion to taxogen concentration (or taxogen partial pressure) has been observed to favor termination of reaction by capping with the ligand split from the telogen, while a relatively low ratio favors addition of more than one taxogen unit prior to capping. While this relationship therefore indicates the desirability of an initial ferric salt charge, it has also been found important that the reacting mixture contain a molar excess of phosphorus (V) compound with respect to ferric compound throughout the course of the reaction. Unless there is such an excess, the reaction essentially stops, possibly because iron ions tie up the phosphorus compound in a reaction product or 1:1 complex, thereby precluding further action of the phosphorus compound as a promoter. This reaction product or complex is believed to remain effective as a source of ferric ions which limit the formation of $n \geq 2$ telomerization products but not as a promoter for initiating the olefin-telogen reaction. Because phosphorus compound appears to be depleted over the course of the reaction while the proportion of ferric ion typically grows, the initial charge should contain an excess of phosphorus compound somewhat greater than that needed to sustain the reaction. Given the reactivity of the taxogen and telogen, an appropriate excess can be readily arrived at.

Progress of the reaction depends upon maintaining a supply of both free phosphorus compound and metallic iron throughout the reaction period. In order to assure the continued availability of phosphorus compound and metallic iron, it is, therefore, necessary to control not only the initial phosphorus compound and ferric chloride content but also the overall quantity of metallic iron available for dissolution and also the area of contact between the liquid phase and the source of metallic iron. Intensity of agitation also affects this balance.

For any given system, those skilled in the art may readily arrive at an appropriate combination of these parameters, which are not narrowly critical. Preferably, the system is operated with vigorous agitation and contains a semi-permanent iron source, i.e., a quantity of iron sufficient to provide for several batches (or several multiples of residence time in a continuous system) without significant variation in surface area. Such a system both provides high productivity and facilitates maintenance of an effective supply of both phosphorus compound and metallic iron.

Vigorous agitation is particularly desirable for promotion of mass transfer of taxogen into the liquid phase where the taxogen is in the gaseous state under the conditions prevailing in the reaction. However, agitation is also considered important to promote mass transfer between the bulk liquid phase and the surface of the source of metallic iron. Based on the results observed, dissolution of a measurable amount of iron into the reacting mixture is important to the progress of the reaction. Whether the dissolution of the iron produces ferrous ions which initiate reaction in the bulk liquid phase in accordance with the mechanism postulated above, or whether reaction occurs, for example, at the surface of the source of iron, agitation is considered important for promoting mass transfer between that surface and the bulk liquid.

Generally, the reaction should be carried out at a temperature which is high enough to provide rapid conversion but low enough to avoid thermal degradation of reactants or products and formation of excessive by-products or tars. The optimum reaction temperature may vary with the nature of the taxogen and telogen and, in any event, is not narrowly critical. In the case of the reaction of ethylene and carbon tetrachloride, a preferred reaction temperature is between about 70°C and about 130°C, preferably 80°C to 125°C.

When the taxogen is in the gaseous state at reaction conditions, conversion rate tends to increase with increasing taxogen partial pressure, but so too does the formation of by-products, especially $n \geq 2$ telomerization products. Thus, it is preferred that taxogen pressure be maintained at the lowest level consistent with good productivity. In the case of the reaction of ethylene with carbon tetrachloride, satisfactory rates of reaction can be obtained without excessive by-product formation at a gauge pressure in the range of from about $1 \times 10^5$ Pa to about $14 \times 10^5$ Pa (about 1 to about 14 atmospheres). For propylene reactions, the preferred range of reaction pressure is somewhat lower but is not narrowly critical.

Under a given set of reaction conditions, by-produced formation may further be controlled by terminating the reaction short of 100% conversion. However, it has been found that by use of a reaction promotion system comprising a phosphorus (V) compound and a source of metallic iron, very high

conversions can be achieved without significant formation of n≥2 telomerization products or of other by-products. Thus, for example, in the case of the reaction of ethylene and carbon tetrachloride, optimal conversion, that is, conversion with low by-product formation, has been determined to be about 99.3%. Moreover, the optimum is not a sharp one; and industrial process control is facilitated by the existence of an optimal plateau at conversions in the range of about 98.8% to about 99.8%. Similarly high conversions are commercially advantageous for reactions of other telogens and other taxogens in accordance with the process of the invention. However, for all taxogens the reaction should be terminated as soon as practicable after the desired conversion is reached, so as to avoid overreaction and formation of by-products.

The presence of water has been found to have a deleterious effect on the rate of reaction in the process of the invention. Thus, to provide for maximum yields and selectivity, the ferrous salt initially charged to the reaction mixture is preferably anhydrous. Other components of the reaction mixture are preferably also devoid of free water. Preferably, the water content of the liquid phase is maintained at not higher than about 0.5 mole percent during the course of the reaction.

Although the process of the invention is conveniently carried out batchwise, it is also adapted for continuous operation, for example, in a cascaded stirred tank reaction system. While the reacting mixture is less corrosive to certain stainless steels than the phosphite systems of Asahara et al. and Takamizawa et al., it nonetheless attacks various alloys of iron. Consequently, glass lined or nickel alloy reactors should be used. It may be convenient to dispose the source of iron in a separate vessel in an external circulation loop through which the liquid phase reaction mixture is pumped during the reaction.

In many instances the product of the reaction is a substantially single phase material which may be used as an end product or intermediate without the necessity of extensive separation or purification steps. In certain cases, for example, the reaction of carbon tetrahalide and ethylene in the presence of triethyl phosphate, a very minor amount of a lighter second phase separates from the reaction product. In a batch reaction system, this second phase may advantageously be preserved as a heel in the reaction and used as a source of ferric salt and phosphorus compound for the next batch.

Monoadducts of taxogens and telogens are produced by the process of the invention at high yield, high selectivity and high productivity without undue risk of runaway reaction or explosion. Safe operation and effective process control are readily achieved. Formation of tars is essentially avoided. Solvents or catalyst modifiers such as acetonitrile are not needed in the process, so that high reactor payloads can be obtained, further contributing to productivity. For these reasons, and because the process provides the opportunity for use of relatively inexpensive raw materials, the present invention affords a means for highly economical production of 1,1,1,3-tetrachloropropane as well as a large variety of other monoadducts of taxogens and telogens.

The following examples illustrate the invention. Unless otherwise indicated, all percentages are given on a weight basis.

Example 1

Carbon tetrachloride (267 g), ferric chloride (1.00 g), triethyl phosphate (2.74 g), and an iron wire (0.023 cm diameter, 1.43 g) wrapped around a Teflon polymeric fluorocarbon rod (0.64 cm diameter) were placed in a 300 ml Hastelloy C autoclave equipped with a stirrer, an internal cooling coil and an external heating mantle. The autoclave was flushed twice with nitrogen and once with ethylene, then pressurized to approximately $3.4\times10^5$ Pa gauge (3.4 atm gauge) with ethylene and sealed. The mixture contained in the autoclave was stirred at 600 rpm and heated to 120°C, at which temperature the pressure was observed to be approximately $11.0\times10^5$ Pa gage (10.9 atm gauge). As a result of the reaction of carbon tetrachloride with ethylene, the pressure in the autoclave then dropped rapidly. When the pressure dropped below about $9.8\times10^5$ Pa gauge (9.7 atm gauge), the ethylene feed valve was reopened and the autoclave repressurized to about $9.8\times10^5$ Pa gauge (9.7 atm gauge) and maintained at that pressure for a total of 240 minutes, the reactor was then cooled and vented. A product mixture (318 g) was obtained. No tars or solids were produced. However, a slight flowable second phase did separate upon standing. The product mixture was analyzed and found to contain 94.8% by weight of 1,1,1,3-tetrachloropropane. Only 0.3% of carbon tetrachloride remained. The yield based on carbon tetrachloride initially present was 95.6%. The iron wire was weighed, and it was determined that 0.31 g of iron had dissolved in the reaction mixture during the course of the reaction.

Example 2

Using the method and apparatus generally described in Example 1, carbon tetrachloride (263 g) was reacted with ethylene at about $9.8\times10^5$ Pa gauge (9.7 atm gauge) in the presence of initially added tributyl phosphate (2.42 g), ferric chloride (1.00 g) and iron powder (−100 mesh, 1.41 g). A product mixture (315 g) was obtained, from which no second phase separated upon standing. No tars or solids were produced. The reaction required 210 minutes at 120°C to reach completion. The product mixture was analyzed and found to contain 93.9% by weight of 1,1,1,3-tetrachloropropane. Only 0.3% carbon tetrachloride remained. The yield based on carbon tetrachloride initially present was 95.2%.

Example 3

Using the method and apparatus generally described in Example 1, carbon tetrachloride (272 g) was reacted with ethylene at about $6.9\times10^5$ Pa gauge (6.8 atm gauge) in the presence of triethyl phosphate (4.16 g), ferric chloride (1.00 g) and a mild steel rod (surface area 13 $cm^3$). A product mixture (325 g), similar in nature to the product mixture in Example 1, was obtained. The reaction required 326 minutes at 120°C to reach completion. The product mixture was analyzed and found to contain 95.9% by weight of 1,1,1,3-tetrachloropropane. Only 0.9% carbon tetrachloride remained. The yield based on carbon tetrachloride initially present was 96.9%. The mild steel rod was weighed, and it was determined that 0.53 g of iron had dissolved in the reaction mixture during the course of the reaction.

Example 4

Carbon tetrachloride (273 g), triethyl phosphate (4.05 g), ferric chloride (1.03 g), and two mild steel rods having a total surface area of 26 $cm^2$ were charged to a 300 ml Hastelloy C autoclave provided with an internal cooling coil. The autoclave was thereafter flushed twice with nitrogen and once with ethylene, pressurized with ethylene to about $4.1\times10^5$ Pa gauge (4.1 atm gauge), and sealed. The mixture contained in the autoclave was stirred at 600 rpm and heated to 120°C at which temperature the pressure was observed to be approximately $8.3\times10^5$ Pa gauge (8.2 atm gauge). As a result of the reaction of carbon tetrachloride with ethylene, the pressure in the autoclave then dropped rapidly. Within one minute of the reaction mixture reaching 120°C, the ethylene feed valve was reopened and the autoclave pressurized to about $9.8\times10^5$ Pa gauge (9.7 atm gauge) and maintained there for 150 minutes. The reactor was then cooled and vented. A product mixture (327 g), similar in nature to that in Example 1, was obtained. No tars or solids were produced. The product mixture was analyzed and found to contain 95.1% by weight of 1,1,1,3-tetrachloropropane. Only 0.4% carbon tetrachloride remained. The yield based on carbon tetrachloride initially present was 96.4%. The mild steel rods were weighed, and it was determined that 0.54 g of iron had dissolved in the reaction mixture during the course of the reaction. A repeat of this reaction required 190 minutes to reach completion and the yield was 96.6%.

Example 5

Using the method and apparatus generally described in Example 4, reactions run with various phosphoryl compounds, at equivalent molar concentration and under conditions otherwise identical to those described in Example 4, resulted in the yields, reaction times and product appearance as shown in the table below:

| Phosphoryl compound | | Reaction time (min) | yield[a] | Conversion | Product appearance |
|---|---|---|---|---|---|
| $OP(OEt)_3$ | | 150 | 96.4% | 99.6% | No tars |
| | | 190 | 96.6% | 99.7% | no solids. slight flowable second phase |
| $OP(OBu^n)_3$ | | 184 | 95.3% | 99.2% | One clear phase, no tars, no solids |
| $OP(OMe)_2(me)$ | | 304 | 93.4% | 99.5% | Solids, tars |
| $OP(OEt)_2(CH=CH_2)$ | | 253 | 95.4% | 99.7% | No tars no solids cloudy product |
| $OP(OEt)_2(CH_2CO_2Et)$ | | 125 | 96.9% | 99.5% | Like $OP(OEt)_3$ |
| $OP(OEt)_2(H)$ | | 555 | 92.5% | 95.6% | Solids, tars |
| $OP(OBu^n)_2(H)$ | | 313 | 92.9% | 99.1% | No tars, no solids, cloudy product |
| $OP(OBu^n)(OH)_2$ / $OP(OBu^n)_2OH$ mixture | 45/55 | 490 | 89.8% | 95.9% | No tars, slight solids |
| $OP(OEt)_2OH$ | | 552 | 92.8% | 75.3% | No tars, no solids |

(a) Yield is based on $CCl_4$ initially charged unless conversion is less than 98.5%, in which case the yields is based on $CCl_4$ consumed.

Example 6

Using the method and apparatus generally described in Example 4, reactions run with different added amounts of water, under conditions otherwise identical to those described in Example 4, resulted in the yields, reaction times, and product appearance as shown in the table below:

| Water level | Yield | Reaction time | Product appearance |
|---|---|---|---|
| 0 | 96.4%, 96.6% | 150, 190 min | No tars, no solids |
| 183 ppm | 96.2% | 149 min | No tars, no solids |
| 544 ppm | 95.9% | 195 min | No tars, no solids |
| 1640 ppm | 95.6% | 230 min | Slight tars, slight solids |
| 2520 ppm | 95.4% | 263 min | Slight tars, slight solids |
| 5600 ppm | 92.5% | 545 min | Tars, solids |

Example 7

Using the method and apparatus generally described in Example 4, reactions run at different stirring rates under conditions otherwise identical to those described in Example 4, resulted in the yields and reaction times shown in the table below:

| Stirrer rpm | Yield | Conversion | Reaction time | Iron dissolved |
|---|---|---|---|---|
| 600 | 96.4% | 99.6% | 150 min | 0.54 g |
| 600 | 96.6% | 99.7% | 190 min | 0.58 g |
| 1000 | 96.5% | 99.5% | 134 min | 0.56 g |
| 1000 | 96.3% | 99.7% | 124 min | 0.59 g |

Example 8

Using the method and apparatus generally described in Example 4, reactions run at different temperatures, under conditions otherwise identical to those described in Example 4, resulted in the yields and reaction times set forth in the table below:

| Temperature | Reaction time | Conversion | Yield |
|---|---|---|---|
| 120°C | 150, 190 min | 99.6%, 99.7% | 96.4%, 96.6% |
| 100°C | 240 min | 99.5% | 96.8% |
| 80°C | 446 min | 91.9% | 97.0% |

Example 9

Carbon tetrachloride (271 g), ferric chloride (1.00 g), triethyl phosphate (5.90 g) and four mild steel rods having a total surface area of 52 $cm^2$ were charged to the autoclave described in Example 1. The mixture was thereafter flushed twice with nitrogen and once with ethylene, pressurized to $4.1 \times 10^5$ Pa gauge (4.1 atm gauge) and sealed. The mixture contained in the autoclave was stirred at 1000 rpm and heated to 80°C, at which temperature the pressure was observed to be approximately $8.3 \times 10^5$ Pa gauge (8.2 atm gauge). As a result of the reaction of carbon tetrachloride with ethylene, the pressure dropped rapidly. When the pressure dropped to less than about $6.9 \times 10^5$ Pa gauge (6.8 atm gauge), the ethylene feed valve was reopened and the autoclave repressurized to about $6.9 \times 10^5$ Pa gauge (6.8 atm gauge) and maintained at that pressure for 484 minutes. The reactor was then cooled and vented. A product mixture (325 g), similar in nature to that in Example 1, was obtained. No tars or solids were produced. The product mixture was analyzed and found to contain 94.9% by weight of 1,1,1,3-tetrachloropropane. Only 0.9% carbon tetrachloride remained. The yield based on carbon tetrachloride initially present was 96.4%. During the course of the reaction 1.08 g of iron dissolved from the mild steel rods.

Example 10

Using the method and apparatus generally described in Example 4, carbon tetrachloride (278 g), triethyl phosphate (4.05 g), and two mild steel rods having a total surface area of 26 $cm^2$ were charged to the autoclave. The mixture was thereafter flushed twice with nitrogen and once with ethylene, pressurized with ethylene to about $3.4\times10^5$ Pa gauge (3.4 atm gauge) and sealed. The mixture in the autoclave was stirred at 600 rpm and heated to 120°C, at which temperature the pressure was observed to be approximately $9.6\times10^5$ Pa gauge (9.5 atm gauge). As a result of reaction of the carbon tetrachloride with ethylene, the pressure in the autoclave dropped rapidly. When the pressure dropped below about $6.9\times10^5$ Pa gauge (6.8 atm gauge), the ethylene feed valve was reopened, the autoclave repressurized to about $6.9\times10^5$ Pa gauge (6.8 atm gauge) and then maintained at that pressure for 240 minutes. The reactor was then cooled and vented. A product mixture (331 g), similar in nature to that in Example 1, was obtained. No tars or solids were produced. The product mixture was analyzed and found to contain 93.5% by weight of 1,1,1,3-tetrachloropropane. Only 0.6% carbon tetrachloride remained. The yield based on carbon tetrachloride initially present was 94.2%. During the course of the reaction 0.81 g of iron dissolved from the mild steel rods.

Example 11

Carbon tetrachloride (265 g), triethyl phosphate (4.18 g), and two mild steel rods having a total surface area of 26 $cm^2$ were charged to the autoclave described in Example 1. The autoclave was thereafter flushed three times with nitrogen and sealed. The mixture contained in the autoclave was stirred at 600 rpm and heated to 120°C, at which point the pressure was approximately $3.2\times10^5$ Pa gauge (3.2 atm gauge). After the mixture had been heated at 120°C for 37 minutes, the ethylene feed valve was opened and the autoclave pressurized to about $6.9\times10^5$ Pa gauge (6.8 atm gauge) and maintained at that pressure for 280 minutes. After 208 minutes of ethylene addition, 1.07 g of additional triethyl phosphate was charged to the autoclave, resulting in an increased reaction rate at that point. Substantially complete reaction was effected after a total of 280 minutes of ethylene addition. The reactor was cooled and vented. A product mixture (317 g), similar in nature to that in Example 1, was obtained. No tars or solids were produced. The product mixture upon analysis was found to contain 94.9% by weight of 1,1,1,3-tetrachloropropane. Only 0.4% carbon tetrachloride remained. The yield based on carbon tetrachloride initially present was 96.2%. The mild steel rods were weighed and it was determined that 0.95 g of iron had dissolved in the reaction mixture during the course of the reaction.

Example 12

Using the method and apparatus generally described in Example 11, carbon tetrachloride (270 g), triethyl phosphate (4.03 g), and two mild steel rods having a total surface area of 26 $cm^2$ were heated for 60 minutes at 120°C under nitrogen. The autoclave was then pressurized to about $9.8\times10^5$ Pa gauge (9.7 atm gauge) with ethylene and maintained there for 285 minutes. Additional triethyl phosphate was not added. The reactor was cooled and vented. A product mixture (323 g), similar in nature to that in Example 1, was obtained. No tars or solids were produced. The product mixture was analyzed and found to contain 94.5% by weight of 1,1,1,3-tetrachloropropane. Only 0.9% carbon tetrachloride remained. The yield based on carbon tetrachloride initially present was 95.4%. During the course of the reaction 0.86 g of iron from the mild steel rods dissolved. Similarly, a reaction run at 110°C with an initial hold time of 95 minutes required 261 minutes of ethylene addition and resulted in a yield of 95.6%.

Example 13

Using the method and apparatus generally described in Example 11, carbon tetrachloride (271 g), a 1% ferric chloride in triethyl phosphate solution (4.04 g) and a mild steel rod having a surface area of 13 $cm^2$ were heated for 60 minutes at 120°C under nitrogen. The auto-clave was then pressurized to about $8.7\times10^5$ Pa gauge (8.6 atm gauge) with ethylene and maintained at that pressure for 360 minutes. Additional triethyl phosphate was not added during the course of this reaction. The reactor was cooled and vented. A product mixture (234 g), similar in nature to that in Example 1, was obtained. This product was analyzed and found to contain 94.7% by weight of 1,1,1,3-tetrachloropropane. Only 0.3% carbon tetrachloride remained. The yield based on carbon tetrachloride initially present was 95.5%. During the course of the reaction 0.70 g of iron dissolved from the mild steel rod. Similarly, the following results were obtained with a 1% ferric chloride in triethyl phosphate solution:

| Mild steel rods | Pressure (×$10^5$ Pa gauge) | Initial hold time | Ethylene addition time | Conversion | Yield |
|---|---|---|---|---|---|
| 2 | 6.9 | 25 min | 223 min | 98.8% | 95.9% |
| 2 | 6.9 | 25 min | 467 min | 99.9% | 95.2% |
| 2 | 6.9 | 27 min | 432 min[a] | 99.7% | 96.0% |
| 1 | 9.8 | 60 min | 241 min | 99.6% | 94.6% |
| 1 | 9.8 | 63 min | 370 min | 99.3% | 95.2% |

(a) 1.07 g of $OP(OEt)_3$ added after 252 minutes of ethylene addition, allowing early completion.

Example 14

Using the method and apparatus generally described in Example 13, the following results were obtained using a 5% triethyl phosphite in triethyl phosphate solution:

| Mild steel rods | Pressure (×$10^5$ Pa gauge) | Initial hold time | Ethylene addition time | Conversion | Yield |
|---|---|---|---|---|---|
| 2 | 6.9 | 26 min | 409 min[a] | 99.3% | 96.0% |
| 1 | 9.8 | 64 min | 350 min | 99.4% | 95.5% |

(a) 1.07 g $OP(OEt)_3$ added after 285 minutes of ethylene addition, allowing early completion.

Example 15

Carbon tetrachloride (258 g), triethyl phosphate (3.67 g), ferric chloride (1.01 g) and one mild steel rod with a surface area of 13 $cm^2$ were charged to a 300 ml Hastelloy C autoclave provided with an internal cooling coil. The autoclave was thereafter flushed three times with nitrogen and sealed. The mixture contained in the autoclave was stirred at 600 rpm and heated to 120°C, at which temperature the pressure was observed to be approximately $3.4\times10^5$ Pa gauge (3.4 atm gauge). When the temperature reached 120°C, a propylene feed valve was opened and the autoclave pressurized to about $6.1\times10^5$ Pa gauge (6.1 atm gauge) and maintained at that pressure for 98 minutes. The reactor was then cooled and vented. A product mixture (338 g) was obtained. This mixture was analyzed and found to contain 87.5% by weight of 1,1,1,3-tetrachlorobutane and 3.79% by weight of 2.-methyl-1,1,1,3-tetrachloropropane. Only 1.2% carbon tetrachloride remained. The yield based on the carbon tetrachloride consumed was 95.2%. The mild steel rod was weighed and it was determined that 0.57 g of iron had dissolved during the course of the reaction.

Example 16

1,1,1,3-tetrachloropropane (218 g, 95.6% by weight), ferric chloride (2.98 g), triethyl phosphate (17.6 g) and seven mild steel rods having a total approximate surface area of 90 $cm^2$ were charged to the autoclave described in Example 1. The autoclave was thereafter flushed twice with nitrogen and once with ethylene, pressurized to about $10.3\times10^5$ Pa gauge (10.2 atm gauge) with ethylene, and sealed. The mixture contained in the autoclave was stirred at 600 rpm and heated to 120°C, at which temperature the pressure was observed to be approximately $17.2\times10^5$ Pa gauge (17.0 atm gauge). As a result of the reaction of the ethylene with 1,1,1,3-tetrachloropropane, the pressure in the autoclave then dropped rapidly. Two minutes after reaching 120°C, the ethylene feed valve was reopened and the autoclave repressurized to about $17.2\times10^5$ Pa gauge (17.0 atm gauge) and maintained at that pressure for 191 minutes. The reactor was then cooled and vented. A product mixture (274 g) was obtained. No tars were formed, but the entire mixture solidified upon standing. The mixture was warmed slightly to approximately 50°C whereupon the mixture liquified and was poured from the reactor. The mixture was analyzed and found to contain 82.9% by weight of 1,3,3,5-tetrachloropentane. Only 0.9% 1,1,1,3-tetrachloropropane remained. The yield based on 1,1,1,3-tetrachloropropane consumed was 95.5%. 1,3,3,5-tetrachloropentane has a literature melting point of 32.5°C to 34.0°C. The mild steel rods were weighed and it was determined that 2.43 g of iron had dissolved into the reaction mixture during the course of the reaction.

Example 17

Using the method and apparatus generally described in Example 4, reactions run with different metallic iron sources, under conditions otherwise identical to those described in Example 4, resulted in the yields, conversions and reaction times set forth in the table below:

| Iron source | (cm$^2$) surface area | Reaction time (minutes) | Conversion (%) | Yield (%) |
|---|---|---|---|---|
| Pure iron bars | 22 | 93, 97 | 99.5, 99.6 | 95.7, 95.8 |
| Carbon steel bars | 19 | 130, 131 | 99.1, 99.4 | 95.9, 96.2 |
| Carbon steel rods | 26 | 91, 97 | 98.8, 99.3 | 96.0, 95.6 |
| Mild steel rods | 26 | 150, 190 | 99.6, 99.7 | 96.4, 96.6 |
| Stainless steel 304 bars | 20 | 61 | 0.0 | 0.0 |
| Stainless steel 316 bars | 17 | 292 | 0.0 | 0.0 |
| Stainless steel 410 bars | 20 | 218 | 99.5 | 95.7 |
| Stainless steel 442 bars | 22 | 400 | 58.5 | 97.0[a] |
| Stainless steel 501 bars | 19 | 70 | 0.0 | 0.0 |
| Carbon steel/ molybdenum[b] | 21 | 130 | 99.3 | 96.1 |
| Carbon steel/ nickel[c] | 21 | 273 | 99.4 | 95.7 |
| Cast iron bars (1st run) | 19 | 197 | 99.5 | 95.8 |
| Cast iron bars (2nd run)[d] | 19 | 330 | 99.1 | 95.7 |
| Cast iron bars (3rd run)[d] | 19 | 423 | 95.5 | 96.9[a] |

(a) Yield based on $CCl_4$ consumed.
(b) 0.13% C, 0.74% Mn, 0.014% P, 0.017% S, 0.20% Si, 0.48% Mo
(c) 0.14% C, 0.010% P, 0.011% S, 0.20% Mn, 0.19% Si, 2.96% Ni, 1.70% Cr, 0.41% Mo, 0.16% Cu, 0.001% V.
(d) Black scale left on surface after reaction, not removed between runs.

Example 18

Using the method and apparatus generally described in Example 4, a reaction run under an ethylene pressure of $6.9 \times 10^5$ Pa gauge (6.8 atm gauge), under conditions otherwise identical to those described in Example 4, required 254 minutes to reach 99.5% conversion with a yield of 96.3% A similar reaction using only one mild steel rod required 326 minutes to reach 99.1% conversion with a yield of 97.0%.

## Claims

1. A process for preparing a monoadduct of a taxogen and a telogen reactive with said taxogen, comprising reacting said telogen and said taxogen in the presence of both a source of metallic iron that is effective as an activator for the reaction, and a promoter for the reaction, said promoter comprising a phosphorus (V) compound that contains a phosphoryl group.
2. A process as set forth in Claim 1 wherein said taxogen comprises an olefin.
3. A process as set forth in Claim 2 wherein said olefin comprises a hydrocarbon.
4. A process as set forth in Claim 1 wherein said telogen comprises an alkyl halide.
5. A process as set forth in Claim 4 wherein said alkyl halide contains a trihalomethyl or a terminal diahalomethyl group.

6. A process as set forth in Claim 1 wherein said telogen is selected from the group consisting of carbon tetrahalides and haloforms.

7. A process as set forth in Claim 1 wherein said phosphorus compound is selected from the group consisting of phosphate esters, phosphorus pentoxide, phosphoryl halides, and those phosphoryl compounds having the structure

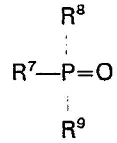

where $R^7$ is a substituted or unsubstituted hydrocarbyl group and $R^8$ and $R^9$ are independently selected from the group consisting of hydroxy, alkoxy, aryloxy, and substituted or unsubstituted hydrocarbyl.

8. A process as set forth in Claim 7 wherein said phosphorus compound is selected from the group consisting of alkyl phosphates and alkyl phosphonates.

9. A process as set forth in Claim 1 wherein said source of metallic iron is selected from the group consisting of carbon steel, cast iron and wrought iron.

10. A process as set forth in Claim 1 wherein said source of metallic iron comprises mild steel.

11. A process for preparing a monoadduct of a taxogen and a telogen reactive with said taxogen while substantially avoiding the formation of higher telomerization products, the process comprising reacting said telogen and an olefin in a reaction system comprising a liquid phase and a source of metallic iron in contact with said liquid phase, said source of metallic iron being effective as an activator for said reaction, said liquid phase comprising said telogen and a promoter for the reaction, said promoter comprising a phosphorus (V) compound that contains a phosphoryl group and is compatible with said telogen.

12. A process as set forth in Claim 11 wherein a reaction system is prepared comprising a liquid phase containing said telogen and said phosphorus (V) compound in contact with said source of metallic iron, and said olefin is thereafter introduced into said system and reacted with said telogen.

13. A process as set forth in Claim 12 wherein a ferric salt is dissolved in said liquid phase prior to initiation of said reaction.

14. A process as set forth in Claim 13 wherein said source of metallic iron is heated in the presence of said telogen and said promoter to produce said ferric salt prior to the introduction of said olefin.

15. A process as set forth in Claim 13 wherein said ferric salt is substantially anhydrous.

16. A process as set forth in Claim 13 wherein said phosphorus (V) compound is initially present in molar excess with respect to said ferric salt.

17. A process as set forth in Claim 16 wherein:

a liquid mixture is prepared containing carbon tetrahalide, said phosphorus (V) compound and ferric chloride;

a gas comprising an olefin selected from the group consisting of ethylene and propylene is introduced into said liquid mixture; and

said olefin is reacted with carbon tetrahalide in the presence of said source of metallic iron to produce said monoadduct.

18. A process as set forth in Claim 17 wherein said olefin comprises ethylene and said monoadduct comprises 1,1,1,3-tetrahalopropane.

19. A process as set forth in Claim 17 wherein said liquid mixture is prepared by mixing carbon tetrachloride, said phosphorus compound and anhydrous ferric chloride.

20. A process as set forth in Claim 17 wherein said olefin comprises ethylene and said telogen comprises carbon tetrachloride.

21. A process as set forth in Claim 17 wherein an anhydrous ferric salt is dissolved in said liquid phase.

**Patentansprüche**

1. Verfahren zum Herstellen eines Monoaddukts eines Taxogens und eines mit dem genannten Taxogen reaktionsfähigen Telogens, welches das Umsetzen des genannten Telogens und des genannten Taxogens in Anwesenheit sowohl einer Quelle von metallischem Eisen, das als Aktivator für die Reaktion wirksam ist, als auch eines Promoters für die Reaktion umfaßt, wobei der genannte Promoter eine Phosphor (V) verbindung umfaßt, die eine Phosphorylgruppe enthält.

2. Verfahren nach Anspruch 1, worin das genannte Taxogen ein Olefin umfaßt.

3. Verfahren nach Anspruch 2, worin das genannte Olefin einen Kohlenwasserstoff umfaßt.

4. Verfahren nach Anspruch 1, worin das genannte Telogen ein Alkylhalogenid umfaßt.

5. Verfahren nach Anspruch 4, worin das genannte Alkylhalogenid eine Trihalogenmethyl- oder eine endständige Dihalogenmethylgruppe enthält.

6. Verfahren nach Anspruch 1, worin das genannte Telogen aus der Gruppe bestehend aus Tetrahalogenkohlenstoffen und und Haloformen ausgewählt wird.

7. Verfahren nach Anspruch 1, worin die genannte Phosphorverbindung aus der Gruppe bestehend

aus Phosphatestern, Phosphorpentoxid, Phosphorylhalogeniden und jenen Phosphorylverbindungen der Formel

$$R^7\text{—}\underset{R^9}{\overset{R^8}{P}}=O$$

worin $R^7$ eine substituierte oder unsubstituierte Hydrocarbylgruppe bedeutet und $R^8$ und $R^9$ unabhängig voneinander ausgewählt sind aus der Gruppe bestehend aus Hydroxy, Alkoxy, Aryloxy und substituiertem oder unsubstituiertem Hydrocarbyl, ausgewählt wird.

8. Verfahren nach Anspruch 7, worin die genannte Phosphorverbindung aus der Gruppe bestehend aus Alkylphosphaten und Alkylphosphonaten ausgewählt wird.

9. Verfahren nach Anspruch 1, worin die genannte Quelle von metallischem Eisen aus der Gruppen bestehend aus Kohlenstoffstahl, Gußeisen und Schmiedeeisen ausgewählt wird.

10. Verfahren nach Anspruch 1, worin die genannte Quelle von metallischem Eisen Weichstahlt ist.

11. Verfahren zum Herstellen eines Monoaddukts eines Taxogens und eines mit dem genannten Taxogen reaktionsfähigen Telogens, wobei die Bildung höherer Telomerisationsprodukte im wesentlichen vermieden wird, welches Verfahren das Umsetzen des genannten Telogens und eines Olefins in einem Reaktionssystem umfassend eine flüssige Phase und eine Quelle von metallischem Eisen in Berührung mit dieser flüssigen Phase umfaßt, wobei die genannte Quelle von metallischem Eisen als Aktivator für die genannte Reaktion wirksam ist, die genannte flüssige Phase das genannte Telogen und einen Promotor für die Reaktion aufweist, welcher Promoter eine Phosphor(V)verbindung umfaßt, die eine Phosphorylgruppe enthält und mit dem genannten Telogen verträglich ist.

12. Verfahren nach Anspruch 11, worin ein Reaktionssystem umfassend eine flüssige Phase, die das genannte Telogen und die genannte Phosphor(V)verbindung in Berührung mit der genannten Quell des metallischen Eisens enthält, hergestellt wird und das genannte Olefin danach in das genannte System eingeführt und mit dem genannten Telogen umgesetzt wird.

13. Verfahren nach Anspruch 12, worin ein Eisen(III)salz in der genannten flüssigen Phase vor Initierung der genannten Reaktion gelöst wird.

14. Verfahren nach Anspruch 13, worin die genannte Quelle des metallischen Eisens in Anwesenheit des genannten Telogens und des genannten Promotors erhitzt wird, um das genannte Eisen(III)salz von Einführung des genannten Olefins zu bilden.

15. Verfahren nach Anspruch 13, worin das genannte Eisen(III)salz im wesentlich wasserfrei ist.

16. Verfahren nach Anspruch 13, worin die genannte Phosphor(V)verbindung anfangs im molarem Überschuß in bezug auf das genannte Eisen(III)salz vorhanden ist.

17. Verfharen nach Anspruch 16, worin

eine flüssige Mischung hergestellt wird, die Tetrahalogenkohlenstoff, die genannte Phosphor(V)verbindung und Eisen(III)chlorid enthält;

ein Gas, das ein Olefin ausgewählt aus der Gruppe bestehnd aus Äthylen und Propylen umfaßt, in die genannte flüssige Mischung eingeführt wird; und

das genannte Olefin mit Tetrahalogenkohlenstoff in Anwesenheit der genannten Quelle metallischen Eisens unter Bildung des genannten Monoaddukts umgesetzt wird.

18. Verfahren nach Anspruch 17, worin das genannte Olefein Äthylen umfaßt und das genannte Monoaddukt 1,1,1,3-Tetrahlogenpropan umfaßt.

19. Verfahren nach Anspruch 17, worin die genannte Flüssige Mischung durch Mischen von Tetrachlorokohlenstoff, der genannten Phosphorverbindung und wasserfreiem Eisen(III)chlorid hergestellt wird.

20. Verfahren nach Anspruch 17, worin das genannte Olefin Äthylen umfaßt und das genannte Telogen Tetrachlorkohlenstoff umfaßt.

21. Verfahren nach Anspruch 17, worin ein wasserfreies Eisen(III)salz in der genannten flüssigen Phase gelöst wird.

**Revendications**

1. Procédé de préparation d'un produit de monoaddition d'un taxogène et d'un télogène réactif avec ce taxogène, consistant à faire réagir ce télogène et ce taxogène en présence à la fois d'une source de fer métallique qui est efficace comme activateur pour la réaction, et d'un agent d'amorçage pour la réaction, cet agent d'amorçage comprenant un composé du phosphore (V) qui contient un groupe phosphoryle.

2. Procédé selon la revendication 1, dans lequel ce taxogène comprend une oléfine.

3. Procédé selon la revendication 2, dans lequel cette oléfine comprend un hydrocarbure.

4. Procédé selon la revendication 1, dans lequel ce télogène comprend un halogènure d'alkyle.

5. Procédé selon la revendication 4, dans lequel cet halogènure d'alkyle contient un groupe trihalométhyle ou un groupe dihalométhyle terminal.

6. Procédé selon la revendication 1, dans lequel ce télogène est choisi dans le groupe constitué de tétrahalogénures de carbone et des haloformes.

7. Procédé selon la revendication 1, dans lequel ce composé du phosphore est choisi dans le groupe constitué des esters phosphoriques, du pentoxyde de phosphore, des halogènures de phosphoryle et de composés phosphorylés répondant à la formule:

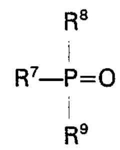

dans laquelle $R^7$ est un groupe hydrocarbyle substitué ou non substitué et $R^8$ et $R^9$ sont choisis indépendamment dans le groupe constitué des groupes hydroxy, alcoxy, aryloxy et hydrocarbyle substitué ou non substitué.

8. Procédé selon la revendication 7, dans lequel ce composé du phosphore est choisi dans le groupe constitué des phosphates d'alkyle et des phosphonates d'alkyle.

9. Procédé selon la revendication 1, dans lequel cette source de fer métallique est choisie dans le groupe constitué de l'acier au carbone, de la fonte et du fer ductile.

10. Procédé selon la revendication 1, dans lequel cette source de fer métallique comprend de l'acier doux.

11. Procédé pour préparer un produit de mono-addition d'un taxogène et d'un télogène réactif avec ce taxogène, tout en évitant dans une large mesure la formation de produits de télomérisation supérieurs, le procédé consistant à faire réagir ce télogène et une oléfine dans un système réactionnel comprenant une phase liquide et une source de fer métallique en contact avec cette phase liquide, cette source de fer métallique étant efficace comme activateur pour cette réaction, cette phase liquide comprenant ce télogène et un agent d'amorçage pour la réaction, cet agent d'amorçage comprenant un composé du phosphore (V) qui contient un groupe phosphoryle et est compatible avec ce télogène.

12. Procédé selon la revendication 11, dans lequel on prépare un système réactionnel comprenant une phase liquide contenant ce télogène et ce composé du phosphore (V) en contact avec cette source de fer métallique, et cette oléfine est ensuite introduite dans ce système et mise à réagir avec ce télogène.

13. Procédé selon la revendication 12, dans lequel un sel ferrique est dissous dans cette phase liquide avant l'amorçage de cette réaction.

14. Procédé selon la revendication 13, dans lequel cette source de fer métallique est chauffée en présence de ce télogène et de cet agent d'amorçage pour produire ce sel ferrique avant l'introduction de cette oléfine.

15. Procédé selon la revendication 13, dans lequel ce sel ferrique est pratiquement anhydre.

16. Procédé selon la revendication 13, dans lequel ce composé du phosphore (V) est initialement présent en excès molaire par rapport à ce sel ferrique.

17. Procédé selon la revendication 16, dans lequel:

— un mélange liquide est préparé, contenant un tétrahalogénure de carbone, ce composé du phosphore (V) et du chlorure ferrique;

— un gaz comprenant une oléfine choisie dans le groupe constitué de l'éthylène et du propylène est introduit dans ce mélange liquide, et

— cette oléfine est mise à réagir avec un tétrahalogénure de carbone en présence de cette source de fer métallique pour produire ce produit de mono-addition.

18. Procédé selon la revendication 17, dans lequel cette oléfine comprend de l'éthylène et ce produit de mono-addition comprend du 1,1,1,3-tétrahalopropane.

19. Procédé selon la revendication 17, dans lequel ce mélange liquide est préparé en mélangeant du tétrachlorure de carbone, ce composé du phosphore et du chlorure ferrique anhydre.

20. Procédé selon la revendication 17, dans lequel cette oléfine comprend de l'éthylène et ce télogène comprend du tétrachlorure de carbone.

21. Procédé selon la revendication 17, dans lequel un sel ferrique anhydre est dissous dans cette phase liquide.